# EUROPÄISCHE PATENTSCHRIFT

(11) **EP 1 719 818 B1**
(45) Veröffentlichungstag und Bekanntmachung des Hinweises auf die Patenterteilung: **17.02.2010**
(21) Anmeldenummer: 06112403.8
(22) Anmeldetag: 10.04.2006
(51) Int. Cl.: C12N 15/62, C12P 13/08

(54) **Verfahren zur Herstellung von L-Aminosäuren unter Verwendung von Stämmen der Familie Enterobacteriaceae mit verstärkter Expression von ytfQ-ORF**
Method for the preparation of L-amino acids using strains of the enterobacteriaceae family with increased expression of ytfQ-ORF
Procédé de préparation de L-amino-acides au moyen de souches de la famille enterobacteriaceae contenant un gène amplifié ytfQ-ORF

(30) Priorität: 03.05.2005 DE 102005020537
(43) Veröffentlichungstag der Anmeldung: 08.11.2006
(73) Patentinhaber: Evonik Degussa GmbH, 45128 Essen (DE)
(72) Erfinder: Dusch, Nicole, Dr., 33824 Werther (DE)

(56) Entgegenhaltungen:
- WO-A-03/008605
- WO-A-03/008606
- US-A- 4 278 765
- WICK L.M. ET AL.,: "short- and long-term chnages in proteome composition and kinetic properties in a culture of escherichia coli during transition from glucose-excess to glucose-limited growth conditions in continous culture and vice versa" ENVIRONM. MICROBIOLOGY, Bd. 3, Nr. 9, 2001, Seiten 588-599, XP002393093
- RAMAN B. ET AL.,: "proteome analysis to assess physiological changes in escherichia coli grown under glucose-limited fed-batch conditions" BIOTECHNOLOGY AND BIOENGINEERING, Bd. 92, Nr. 3, 5. November 2005 (2005-11-05), Seiten 384-392, XP002393094

### SEQUENCE LISTING

<110> Degussa AG
<120> Verfahren zur Herstellung von L-Aminosäuren unter Verwendung von verbesserten Stämmen der Familie Enterobacteriaceae
<130> 050063 BT
<160> 8
<170> PatentIn version 3.3
<210> 1
   <211> 957
   <212> DNA
   <213> Escherichia coli
<220>
   <221> CDS
   <222> (1)..(957)
   <223> ytfQ Kodierregion
<400> 1
   atg tgg aaa cgc tta ctt ata gtc tct gca gtc tcg gca gcc atg tcg        48
   Met Trp Lys Arg Leu Leu Ile Val Ser Ala Val Ser Ala Ala Met Ser
   1               5                   10                  15
   tct atg gcg ttg gcc gct cca tta acc gtt gga ttt tcg cag gtc gga        96
   Ser Met Ala Leu Ala Ala Pro Leu Thr Val Gly Phe Ser Gln Val Gly
               20                  25                  30
   tcg gaa tca ggc tgg cgt gcc gca gaa acc aat gtg gcg aaa agt gaa       144
   Ser Glu Ser Gly Trp Arg Ala Ala Glu Thr Asn Val Ala Lys Ser Glu
           35                  40                  45
   gcc gaa aag cgc gga atc acg ttg aaa att gcc gat ggt cag caa aag       192
   Ala Glu Lys Arg Gly Ile Thr Leu Lys Ile Ala Asp Gly Gln Gln Lys
       50                  55                  60
   cag gaa aac cag att aaa gcg gta cgt tcc ttc gtt gca caa ggg gtg       240
   Gln Glu Asn Gln Ile Lys Ala Val Arg Ser Phe Val Ala Gln Gly Val
   65                  70                  75                  80
   gat gcg atc ttt atc gct ccg gtg gtc gcg aca ggt tgg gaa ccg gta       288
   Asp Ala Ile Phe Ile Ala Pro Val Val Ala Thr Gly Trp Glu Pro Val
                   85                  90                  95
   tta aaa gag gcg aaa gat gcc gaa atc ccg gta ttc ttg ctc gat cgt       336
   Leu Lys Glu Ala Lys Asp Ala Glu Ile Pro Val Phe Leu Leu Asp Arg
               100                 105                 110
   tcc att gat gtg aaa gac aaa tct ctc tat atg acc acc gtc act gcc       384
   Ser Ile Asp Val Lys Asp Lys Ser Leu Tyr Met Thr Thr Val Thr Ala
           115                 120                 125
   gac aac atc ctc gaa ggc aag ttg att ggt gac tgg ctg gta aaa gaa       432
   Asp Asn Ile Leu Glu Gly Lys Leu Ile Gly Asp Trp Leu Val Lys Glu
       130                 135                 140
   gtg aat ggc aaa cca tgc aac gtg gtg gag ctg cag ggc acc gtt ggg       480
   Val Asn Gly Lys Pro Cys Asn Val Val Glu Leu Gln Gly Thr Val Gly
   145                 150                 155                 160
   gcc agc gtc gcc att gac cgt aag aaa ggc ttt gcc gaa gcc att aag      528
   Ala Ser Val Ala Ile Asp Arg Lys Lys Gly Phe Ala Glu Ala Ile Lys
                   165                 170                 175
   aat gcg cca aat atc aaa atc atc cgc tcg cag tca ggt gac ttc acc      576
   Asn Ala Pro Asn Ile Lys Ile Ile Arg Ser Gln Ser Gly Asp Phe Thr
               180                 185                 190
   cgc agt aaa ggc aaa gaa gtc atg gag agc ttt atc aaa gcg gaa aac      624
   Arg Ser Lys Gly Lys Glu Val Met Glu Ser Phe Ile Lys Ala Glu Asn
           195                 200                 205
   aac ggc aaa aac atc tgc atg gtt tac gcc cat aac gac gac atg gtg      672
   Asn Gly Lys Asn Ile Cys Met Val Tyr Ala His Asn Asp Asp Met Val
       210                 215                 220
   att ggt gca att cag gca att aaa gaa gcg ggc ctg aaa ccg ggc aaa      720
   Ile Gly Ala Ile Gln Ala Ile Lys Glu Ala Gly Leu Lys Pro Gly Lys
   225                 230                 235                 240
   gat atc ctc acg ggt tcc att gac ggt gta ccg gac atc tac aaa gcg      768
   Asp Ile Leu Thr Gly Ser Ile Asp Gly Val Pro Asp Ile Tyr Lys Ala
                   245                 250                 255
   atg atg gat ggc gaa gcg aac gcc agt gtt gaa ctg acg ccg aat atg      816
   Met Met Asp Gly Glu Ala Asn Ala Ser Val Glu Leu Thr Pro Asn Met
               260                 265                 270
   gca ggt ccc gcc ttc gac gcg ctg gag aaa tac aaa aaa gac ggc acc      864
   Ala Gly Pro Ala Phe Asp Ala Leu Glu Lys Tyr Lys Lys Asp Gly Thr
           275                 280                 285
   atg cct gaa aag ctg acg tta acc aaa tcc acc ctt tac ctg cct gat      912
   Met Pro Glu Lys Leu Thr Leu Thr Lys Ser Thr Leu Tyr Leu Pro Asp
       290                 295                 300
   acc gca aaa gaa gaa tta gag aag aag aaa aat atg ggg tat tga          957
   Thr Ala Lys Glu Glu Leu Glu Lys Lys Lys Asn Met Gly Tyr
   305                 310                 315
<210> 2
   <211> 318
   <212> PRT
   <213> Escherichia coli
<400> 2
   Met Trp Lys Arg Leu Leu Ile Val Ser Ala Val Ser Ala Ala Met Ser
   1               5                   10                  15
   Ser Met Ala Leu Ala Ala Pro Leu Thr Val Gly Phe Ser Gln Val Gly
               20                  25                  30
   Ser Glu Ser Gly Trp Arg Ala Ala Glu Thr Asn Val Ala Lys Ser Glu
           35                  40                  45
   Ala Glu Lys Arg Gly Ile Thr Leu Lys Ile Ala Asp Gly Gln Gln Lys
       50                  55                  60
   Gln Glu Asn Gln Ile Lys Ala Val Arg Ser Phe Val Ala Gln Gly Val
   65                  70                  75                  80
   Asp Ala Ile Phe Ile Ala Pro Val Val Ala Thr Gly Trp Glu Pro Val
   85 90 95
   Leu Lys Glu Ala Lys Asp Ala Glu Ile Pro Val Phe Leu Leu Asp Arg
   100 105 110
   Ser Ile Asp Val Lys Asp Lys Ser Leu Tyr Met Thr Thr Val Thr Ala
   115 120 125
   Asp Asn Ile Leu Glu Gly Lys Leu Ile Gly Asp Trp Leu Val Lys Glu
   130 135 140
   Val Asn Gly Lys Pro Cys Asn Val Val Glu Leu Gln Gly Thr Val Gly
   145 150 155 160
   Ala Ser Val Ala Ile Asp Arg Lys Lys Gly Phe Ala Glu Ala Ile Lys
   165 170 175
   Asn Ala Pro Asn Ile Lys Ile Ile Arg Ser Gln Ser Gly Asp Phe Thr
   180 185 190
   Arg Ser Lys Gly Lys Glu Val Met Glu Ser Phe Ile Lys Ala Glu Asn
   195 200 205
   Asn Gly Lys Asn Ile Cys Met Val Tyr Ala His Asn Asp Asp Met Val
   210 215 220
   Ile Gly Ala Ile Gln Ala Ile Lys Glu Ala Gly Leu Lys Pro Gly Lys
   225 230 235 240
   Asp Ile Leu Thr Gly Ser Ile Asp Gly Val Pro Asp Ile Tyr Lys Ala
   245 250 255
   Met Met Asp Gly Glu Ala Asn Ala Ser Val Glu Leu Thr Pro Asn Met
   260 265 270
   Ala Gly Pro Ala Phe Asp Ala Leu Glu Lys Tyr Lys Lys Asp Gly Thr
   275 280 285
   Met Pro Glu Lys Leu Thr Leu Thr Lys Ser Thr Leu Tyr Leu Pro Asp
   290 295 300
   Thr Ala Lys Glu Glu Leu Glu Lys Lys Lys Asn Met Gly Tyr
   305 310 315
<210> 3
   <211> 957
   <212> DNA
   <213> Erwinia carotovora
<220>
   <221> CDS
   <222> (1) .. (957)
   <223> ytfQ Kodierregion
<400> 3
   atg tac aga cgt tta ctg gta gct gtt gct gta agc acg gca tta tgt      48
   Met Tyr Arg Arg Leu Leu Val Ala Val Ala Val Ser Thr Ala Leu Cys
   1               5                   10                  15
   ggt gcc gta cag gca aaa ccc ttg acg gtt ggg ttt tct cag att ggt      96
   Gly Ala Val Gln Ala Lys Pro Leu Thr Val Gly Phe Ser Gln Ile Gly
               20                  25                  30
   tca gaa tca gga tgg cgc tct gca gag act aaa gtg tcg aag caa gaa     144
   Ser Glu Ser Gly Trp Arg Ser Ala Glu Thr Lys Val Ser Lys Gln Glu
           35                  40                  45
   gca gag aag cgt gga ata acg cta aaa att gct gat gct cag cag aaa     192
   Ala Glu Lys Arg Gly Ile Thr Leu Lys Ile Ala Asp Ala Gln Gln Lys
       50                  55                  60
   caa gag aac cag att aag gct gta cgg tca ttc atc gct cag agt gtt     240
   Gln Glu Asn Gln Ile Lys Ala Val Arg Ser Phe Ile Ala Gln Ser Val
   65                  70                  75                  80
   gat gcc ata ttt atc gca ccg gtc gtt gct aca ggc tgg gca cca gtc     288
   Asp Ala Ile Phe Ile Ala Pro Val Val Ala Thr Gly Trp Ala Pro Val
                   85                  90                  95
   tta cag gaa gct aaa gaa gca aaa atc ccg gta ttc ctg ctt gat aga     336
   Leu Gln Glu Ala Lys Glu Ala Lys Ile Pro Val Phe Leu Leu Asp Arg
               100                 105                 110
   acg att gag gtt agc gat cca tcg ctg tac acc gct gct att gcc tct     384
   Thr Ile Glu Val Ser Asp Pro Ser Leu Tyr Thr Ala Ala Ile Ala Ser
           115                 120                 125
   gac agt gtc tat gaa gga aaa gtg gca ggg gaa tgg ctt gtg aaa gag     432
   Asp Ser Val Tyr Glu Gly Lys Val Ala Gly Glu Trp Leu Val Lys Glu
       130                 135                 140
   gct gca ggt aaa ccg tgt aat gtt gtc gaa ttg cag gga acg gtt ggg     480
   Ala Ala Gly Lys Pro Cys Asn Val Val Glu Leu Gln Gly Thr Val Gly
   145                 150                 155                 160
   gct agc gtt gcg atc aac cgc aag aaa ggg ttt gct gaa ggt ata gct     528
   Ala Ser Val Ala Ile Asn Arg Lys Lys Gly Phe Ala Glu Gly Ile Ala
                   165                 170                 175
   tca gat cca cag ata aaa att att cga tca cag tca gga gac ttt act     576
   Ser Asp Pro Gln Ile Lys Ile Ile Arg Ser Gln Ser Gly Asp Phe Thr
               180                 185                 190
   cgc agt aag ggt aaa gag gtc atg gaa agc ttt att aaa gct gag cag     624
   Arg Ser Lys Gly Lys Glu Val Met Glu Ser Phe Ile Lys Ala Glu Gln
           195                 200                 205
   aac gga aaa aat atc tgc gcg gtt tat gca cat aat gat gat atg gct     672
   Asn Gly Lys Asn Ile Cys Ala Val Tyr Ala His Asn Asp Asp Met Ala
       210                 215                 220
   ata ggt gct att cag gca att aaa gag gca ggc cta aaa cct ggc tca     720
   Ile Gly Ala Ile Gln Ala Ile Lys Glu Ala Gly Leu Lys Pro Gly Ser
   225                     230                     235                     240
   caa ata aaa gtt gtc tct att gat ggt gtt ccc gat att ttc aag gcc      768
   Gln Ile Lys Val Val Ser Ile Asp Gly Val Pro Asp Ile Phe Lys Ala
                   245                     250                     255
   atg atg aat ggt gaa gcc aat gct acc gtt gag tta aca ccc aat atg      816
   Met Met Asn Gly Glu Ala Asn Ala Thr Val Glu Leu Thr Pro Asn Met
               260                     265                     270
   gct gga cct gct ttt gat gct tta tta gcg atg aag aag gac gga aaa      864
   Ala Gly Pro Ala Phe Asp Ala Leu Leu Ala Met Lys Lys Asp Gly Lys
           275                     280                     285
   cag cca gaa aaa ttt att cag aca gag tct cgt tta ttg ctg tcc gat      912
   Gln Pro Glu Lys Phe Ile Gln Thr Glu Ser Arg Leu Leu Leu Ser Asp
       290                     295                     300
   acg gca aaa cag gag tat gaa acc aaa aaa gat ctt ggt tat tga          957
   Thr Ala Lys Gln Glu Tyr Glu Thr Lys Lys Asp Leu Gly Tyr
   305                     310                     315
<210> 4
   <211> 318
   <212> PRT
   <213> Erwinia carotovora
<400> 4
   Met Tyr Arg Arg Leu Leu Val Ala Val Ala Val Ser Thr Ala Leu Cys
   1               5                   10                  15
   Gly Ala Val Gln Ala Lys Pro Leu Thr Val Gly Phe Ser Gln Ile Gly
               20                  25                  30
   Ser Glu Ser Gly Trp Arg Ser Ala Glu Thr Lys Val Ser Lys Gln Glu
           35                  40                  45
   Ala Glu Lys Arg Gly Ile Thr Leu Lys Ile Ala Asp Ala Gln Gln Lys
       50                  55                  60
   Gln Glu Asn Gln Ile Lys Ala Val Arg Ser Phe Ile Ala Gln Ser Val
   65                  70                  75                  80
   Asp Ala Ile Phe Ile Ala Pro Val Val Ala Thr Gly Trp Ala Pro Val
                   85                  90                  95
   Leu Gln Glu Ala Lys Glu Ala Lys Ile Pro Val Phe Leu Leu Asp Arg
               100                 105                 110
   Thr Ile Glu Val Ser Asp Pro Ser Leu Tyr Thr Ala Ala Ile Ala Ser
           115                 120                 125
   Asp Ser Val Tyr Glu Gly Lys Val Ala Gly Glu Trp Leu Val Lys Glu
       130                 135                 140
   Ala Ala Gly Lys Pro Cys Asn Val Val Glu Leu Gln Gly Thr Val Gly
   145                 150                 155                 160
   Ala Ser Val Ala Ile Asn Arg Lys Lys Gly Phe Ala Glu Gly Ile Ala
                   165                 170                 175
   Ser Asp Pro Gln Ile Lys Ile Ile Arg Ser Gln Ser Gly Asp Phe Thr
               180                 185                 190
   Arg Ser Lys Gly Lys Glu Val Met Glu Ser Phe Ile Lys Ala Glu Gln
           195                 200                 205
   Asn Gly Lys Asn Ile Cys Ala Val Tyr Ala His Asn Asp Asp Met Ala
       210                 215                 220
   Ile Gly Ala Ile Gln Ala Ile Lys Glu Ala Gly Leu Lys Pro Gly Ser
   225                 230                 235                 240
   Gln Ile Lys Val Val Ser Ile Asp Gly Val Pro Asp Ile Phe Lys Ala
                   245                 250                 255
   Met Met Asn Gly Glu Ala Asn Ala Thr Val Glu Leu Thr Pro Asn Met
               260                 265                 270
   Ala Gly Pro Ala Phe Asp Ala Leu Leu Ala Met Lys Lys Asp Gly Lys
           275                 280                 285
   Gln Pro Glu Lys Phe Ile Gln Thr Glu Ser Arg Leu Leu Leu Ser Asp
       290                 295                 300
   Thr Ala Lys Gln Glu Tyr Glu Thr Lys Lys Asp Leu Gly Tyr
   305                 310                 315
<210> 5
   <211> 18
   <212> DNA
   <213> Artificial Sequence
<220>
   <223> Primer ytfQ-1
<400> 5
   accgtagccg catttttc 18
<210> 6
   <211> 18
   <212> DNA
   <213> Artificial Sequence
<220>
   <223> Primer ytfQ-2
<400> 6
   aatcggcatc aggcatag 18
<210> 7
   <211> 1152
   <212> DNA
   <213> Corynebacterium glutamicum
<220>
   <221> CDS
   <222> (172)..(1125)
<400> 7
   accgtagccg catttttcat agtgagatga aagcgattac aaacttgtga ttaacgtttt      60
   atttactttt ttgaagtgtg atgtaacgca atctgttaca taacgaattg tctatagttt     120
   tttcgcgaac atcttttaac caataataac taccccgacg aggacaaccc t atg tgg     177
                                                            Met Trp
                                                            1
   aaa cgc tta ctt ata gtc tct gca gtc tcg gca gcc atg tcg tct atg      225
   Lys Arg Leu Leu Ile Val Ser Ala Val Ser Ala Ala Met Ser Ser Met
           5                   10                  15
   gcg ttg gcc gct cca tta acc gtt gga ttt tcg cag gtc gga tcg gaa      273
   Ala Leu Ala Ala Pro Leu Thr Val Gly Phe Ser Gln Val Gly Ser Glu
       20                  25                  30
   tca ggc tgg cgt gcc gca gaa acc aat gtg gcg aaa agt gaa gcc gaa      321
   Ser Gly Trp Arg Ala Ala Glu Thr Asn Val Ala Lys Ser Glu Ala Glu
   35                  40                  45                  50
   aag cgc gga atc acg ttg aaa att gcc gat ggt cag caa aag cag gaa      369
   Lys Arg Gly Ile Thr Leu Lys Ile Ala Asp Gly Gln Gln Lys Gln Glu
                   55                  60                  65
   aac cag att aaa gcg gta cgt tcc ttc gtt gca caa ggg gtg gat gcg      417
   Asn Gln Ile Lys Ala Val Arg Ser Phe Val Ala Gln Gly Val Asp Ala
               70                  75                  80
   atc ttt atc gct ccg gtg gtc gcg aca ggt tgg gaa ccg gta tta aaa      465
   Ile Phe Ile Ala Pro Val Val Ala Thr Gly Trp Glu Pro Val Leu Lys
           85                  90                  95
   gag gcg aaa gat gcc gaa atc ccg gta ttc ttg ctc gat cgt tcc att      513
   Glu Ala Lys Asp Ala Glu Ile Pro Val Phe Leu Leu Asp Arg Ser Ile
       100                 105                 110
   gat gtg aaa gac aaa tct ctc tat atg acc acc gtc act gcc gac aac      561
   Asp Val Lys Asp Lys Ser Leu Tyr Met Thr Thr Val Thr Ala Asp Asn
   115                 120                 125                 130
   atc ctc gaa ggc aag ttg att ggt gac tgg ctg gta aaa gaa gtg aat      609
   Ile Leu Glu Gly Lys Leu Ile Gly Asp Trp Leu Val Lys Glu Val Asn
                   135                 140                 145
   ggc aaa cca tgc aac gtg gtg gag ctg cag ggc acc gtt ggg gcc agc      657
   Gly Lys Pro Cys Asn Val Val Glu Leu Gln Gly Thr Val Gly Ala Ser
               150                 155                 160
   gtc gcc att gac cgt aag aaa ggc ttt gcc gaa gcc att aag aat gcg      705
   Val Ala Ile Asp Arg Lys Lys Gly Phe Ala Glu Ala Ile Lys Asn Ala
           165                 170                 175
   cca aat atc aaa atc atc cgc tcg cag tca ggt gac ttc acc cgc agt      753
   Pro Asn Ile Lys Ile Ile Arg Ser Gln Ser Gly Asp Phe Thr Arg Ser
       180                 185                 190
   aaa ggc aaa gaa gtc atg gag agc ttt atc aaa gcg gaa aac aac ggc      801
   Lys Gly Lys Glu Val Met Glu Ser Phe Ile Lys Ala Glu Asn Asn Gly
   195                 200                 205                 210
   aaa aac atc tgc atg gtt tac gcc cat aac gac gac atg gtg att ggt      849
   Lys Asn Ile Cys Met Val Tyr Ala His Asn Asp Asp Met Val Ile Gly
                   215                 220                 225
   gca att cag gca att aaa gaa gcg ggc ctg aaa ccg ggc aaa gat atc      897
   Ala Ile Gln Ala Ile Lys Glu Ala Gly Leu Lys Pro Gly Lys Asp Ile
               230                 235                 240
   ctc acg ggt tcc att gac ggt gta ccg gac atc tac aaa gcg atg atg      945
   Leu Thr Gly Ser Ile Asp Gly Val Pro Asp Ile Tyr Lys Ala Met Met
           245                 250                 255
   gat ggc gaa gcg aac gcc agt gtt gaa ctg acg ccg aat atg gca ggt      993
   Asp Gly Glu Ala Asn Ala Ser Val Glu Leu Thr Pro Asn Met Ala Gly
       260                 265                 270
   ccc gcc ttc gac gcg ctg gag aaa tac aaa aaa gac ggc acc atg cct     1041
   Pro Ala Phe Asp Ala Leu Glu Lys Tyr Lys Lys Asp Gly Thr Met Pro
   275                 280                 285                 290
   gaa aag ctg acg tta acc aaa tcc acc ctt tac ctg cct gat acc gca     1089
   Glu Lys Leu Thr Leu Thr Lys Ser Thr Leu Tyr Leu Pro Asp Thr Ala
                   295                 300                 305
   aaa gaa gaa tta gag aag aag aaa aat atg ggg tat tgagggttgc          1135
   Lys Glu Glu Leu Glu Lys Lys Lys Asn Met Gly Tyr
               310                 315
   tatgcctgat gccgatt                                                  1152
<210> 8
   <211> 318
   <212> PRT
   <213> Corynebacterium glutamicum
<400> 8
   Met Trp Lys Arg Leu Leu Ile Val Ser Ala Val Ser Ala Ala Met Ser
   1               5                   10                  15
   Ser Met Ala Leu Ala Ala Pro Leu Thr Val Gly Phe Ser Gln Val Gly
               20                  25                  30
   Ser Glu Ser Gly Trp Arg Ala Ala Glu Thr Asn Val Ala Lys Ser Glu
           35                  40                  45
   Ala Glu Lys Arg Gly Ile Thr Leu Lys Ile Ala Asp Gly Gln Gln Lys
       50                  55                  60
   Gln Glu Asn Gln Ile Lys Ala Val Arg Ser Phe Val Ala Gln Gly Val
   65                  70                  75                  80
   Asp Ala Ile Phe Ile Ala Pro Val Val Ala Thr Gly Trp Glu Pro Val
                   85                  90                  95
   Leu Lys Glu Ala Lys Asp Ala Glu Ile Pro Val Phe Leu Leu Asp Arg
               100                 105                 110
   Ser Ile Asp Val Lys Asp Lys Ser Leu Tyr Met Thr Thr Val Thr Ala
           115                 120                 125
   Asp Asn Ile Leu Glu Gly Lys Leu Ile Gly Asp Trp Leu Val Lys Glu
       130                 135                 140
   Val Asn Gly Lys Pro Cys Asn Val Val Glu Leu Gln Gly Thr Val Gly
   145                 150                 155                 160
   Ala Ser Val Ala Ile Asp Arg Lys Lys Gly Phe Ala Glu Ala Ile Lys
                   165                 170                 175
   Asn Ala Pro Asn Ile Lys Ile Ile Arg Ser Gln Ser Gly Asp Phe Thr
               180                 185                 190
   Arg Ser Lys Gly Lys Glu Val Met Glu Ser Phe Ile Lys Ala Glu Asn
           195                 200                 205
   Asn Gly Lys Asn Ile Cys Met Val Tyr Ala His Asn Asp Asp Met Val
       210                 215                 220
   Ile Gly Ala Ile Gln Ala Ile Lys Glu Ala Gly Leu Lys Pro Gly Lys
   225                 230                 235                 240
   Asp Ile Leu Thr Gly Ser Ile Asp Gly Val Pro Asp Ile Tyr Lys Ala
                   245                 250                 255
   Met Met Asp Gly Glu Ala Asn Ala Ser Val Glu Leu Thr Pro Asn Met
               260                 265                 270
   Ala Gly Pro Ala Phe Asp Ala Leu Glu Lys Tyr Lys Lys Asp Gly Thr
           275                 280                 285
   Met Pro Glu Lys Leu Thr Leu Thr Lys Ser Thr Leu Tyr Leu Pro Asp
       290                 295                 300
   Thr Ala Lys Glu Glu Leu Glu Lys Lys Lys Asn Met Gly Tyr
   305                 310                 315

## Beschreibung

Diese Erfindung betrifft ein Verfahren zur Herstellung von L-Threonin, unter Verwendung von rekombinanten Mikroorganismen der Familie Enterobacteriaceae, in denen der offene Leserahmen (ORF) mit der Bezeichnung ytfQ verstärkt, insbesondere überexprimiert wird, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden.

### Aufgabe der Erfindung

Die Erfinder haben sich die Aufgabe gestellt, neue Maßnahmen zur verbesserten fermentativen Herstellung von L-Threonin, bereitzustellen.

### Beschreibung der Erfindung

Gegenstand der Erfindung sind rekombinante Mikroorganismen der Familie Enterobacteriaceae, die einen verstärkten oder überexprimierten offenen Leserahmen ytfQ, der für ein Polypeptid kodiert, welches als putatives Bindeprotein eines ATP-abhängigen Zuckertransporters annotiert ist, oder für dessen Genprodukt kodierende Nukleotidsequenzen enthalten, und die eine verbesserte Fähigkeit zur Bildung und Anreicherung von L-Aminosäuren, insbesondere L-Threonin, zeigen.

Als Ausgangspunkt für den Vergleich dienen jeweils die für den ytfQ-ORF nicht rekombinanten Mikroorganismen, die keinen verstärkten ytfQ-ORF enthalten und an denen die Maßnahmen der Erfindung durchgeführt werden.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 80% oder mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2 und SEQ ID No. 4.

Die genannten Mikroorganismen enthalten verstärkte oder überexprimierte Polynukleotide, ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
wobei die Polynukleotide bevorzugt für ein putatives Bindeprotein eines ATP-abhängigen Zuckertransporters kodieren.

Gegenstand der Erfindung ist ebenso ein Verfahren zur fermentativen Herstellung von L-Threonin, unter Verwendung von rekombinanten Mikroorganismen der Familie Enterobacteriaceae, die insbesondere bereits L-Aminosäuren produzieren, und in denen mindestens der offene Leserahmen (ORF) mit der Bezeichnung ytfQ oder für dessen Genprodukt kodierende Nukleotidsecluenzen verstärkt wird bzw. werden.

Bevorzugt werden die beschriebenen Mikroorganismen eingesetzt.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfinderischen Maßnahmen durchgeführt werden.

Gegenstand der Erfindung ist ein Verfahren zur Herstellung von L-Threonin durch Fermentation von rekombinanten Mikroorganismen der Familie Enterobacteriaceae, **dadurch gekennzeichnet, dass** man
a) die L-Threonin produzierenden Mikroorganismen, in denen man den offenen Leserahmen ytfQ oder für dessen Genprodukt kodierende Nukleotidsequenzen oder Allele verstärkt, insbesondere überexprimiert, in einem Medium kultiviert unter Bedingungen, bei denen die gewünschte L-Aminosäure im Medium oder in den Zellen angereichert wird, und,
b) die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten offenen Leserahmen (ORF) mit der Bezeichnung ytfQ, die ebenfalls Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion oder Konjugation, oder einer Kombination dieser Methoden, mit einem Vektor erzeugt, der den gewünschten ORF, das gewünschte Gen, ein Allel dieses ORFs oder Gens oder Teile davon und/oder einen die Exprimierung des ORFs oder Gens verstärkenden Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Als zum Elternstamm geeignete, insbesondere L-Threonin produzierende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660)

Als zum Elternstamm geeignete L-Threonin produzierende Stämme der Gattung Serratia, insbesondere der Art Serratia marcescens sind beispielsweise zu nennen:
- Serratia marcescens HNr21 (Applied and Environmental Microbiology 38(6) : 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Überexpression des Gens oder offenen Leserahmens (ORF) ytfQ, oder dessen Allelen, eine verbesserter Fähigkeit zur Bildung und Anreicherung von L-Aminosäuren, insbesondere L-Threonin zeigen.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus dem ebenfalls zur Familie Enterobacteriaceae gehörenden Erwinia carotovora ist die Nukleotidsequenz für den ytfQ-ORF ebenso bekannt (Accession No.: NC_004547 (Region: 4740175-4739870).

Der ytfQ-ORF von Escherichia coli K12 wird unter anderem durch folgende Angaben beschrieben:
Das Genprodukt des ytfQ-ORFs ist annotiert als putatives Bindeprotein eines ATP-abhängigen Zuckertransporters. Weiterhin wird es als periplasmatische Bindeprotein-Komponente eines putativen D-Ribose Transport-Proteins der Familie der ABC-Transporter bzw. putativer LACI-Typ Regulator der Transkription bzw. Vorläufer des periplasmatischen Bindeproteins eines ABC-Transporters bezeichnet.
   Accession No.: U00096 (Region: 4447985-4448941)
   Alternativer Genname: b4227

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum ytfQ-ORF von Escherichia coli unter der SEQ ID No. 1 und die bekannten Sequenz zum ytfQ-ORF von Erwinia carotovora unter der SEQ ID No. 3 dargestellt. Die von diesen Leserahmen kodierten Proteine sind als SEQ ID No. 2 und SEQ ID No. 4 dargestellt.

Die in den angegebenen Textstellen beschriebenen offenen Leserahmen können erfindungsgemäß verwendet werden. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des ytfQ-ORFs, welche funktionsneutrale Sinnmutationen enthalten, zählen unter anderem solche, die zu höchstens 40 oder zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1 oder SEQ ID No. 3 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1 oder SEQ ID No. 3 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558) .

Zur Erzielung einer Verstärkung können beispielsweise die Expression der Gene oder offenen Leserahmen oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die ORFs, Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die att-site des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, Ipp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden.

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartölome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens den ytfQ-ORF, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus) .

Es ist ebenfalls möglich, Mutationen, welche die Expression der jeweiligen Gene oder offenen Leserahmen betreffen, durch Sequenzaustausch (Hamilton et al.; Journal of Bacteriology 171: 4617-4622 (1989)), Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin mit Stämmen der Familie Enterobacteriaceae vorteilhaft sein, zusätzlich zur Verstärkung des offenen Leserahmens ytfQ, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So können beispielsweise gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Transhydrogenase kodierenden Gene pntA und pntB (European Journal of Biochemistry 158: 647-653 (1986); WO 95/11985),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),

- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Peduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yibD von Escherichia coli (Accession Number AE000439 des National Center for Biotechnology Information (NCBI, Bethesda, MD, USA), DE102004005836.9),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) ytfR von Escherichia coli (Accession Number NC000913 (Region 4449081-4450583) des National Center for Biotechnology Information (NCBI, Bethesda, MD, USA), der auch unter der Bezeichnung ytfS-ORF bekannt ist,
- das Genprodukt des offenen Leserahmens (ORF) ytfT von Escherichia coli (Accession Number NC000913 (Region 4450594-4451619) des National Center for Biotechnology Information (NCBI, Bethesda, MD, USA), und
- das Genprodukt des offenen Leserahmens (ORF) yjfF von Escherichia coli (Accession Number NC000913 (Region 4451630-4452601) des National Center for Biotechnology Information (NCBI, Bethesda, MD, USA)
verstärkt, insbesondere überexprimiert werden.

Weiterhin kann es für die Produktion von L-Aminosäuren, insbesondere L-Threonin vorteilhaft sein, zusätzlich zur Verstärkung des offenen Leserahmens ytfQ, eines oder mehrere der Gene ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das Genprodukt des offenen Leserahmens (ORF) yjfA von Escherichia coli (Accession Number AAC77180 des National Center for Biotechnology Information (NCBI, Bethesda, MD, USA), WO 02/29080),
- das Genprodukt des offenen Leserahmens (ORF) ytfP von Escherichia coli (Accession Number AAC77179 des National Center for Biotechnology Information (NCBI, Bethesda, MD, USA), WO 02/29080),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist und
- das für die Aspartat Ammonium-Lyase kodierende aspA-Gen (WO 03/008603)
abzuschwächen, insbesondere auszuschalten oder die Expression zu verringern.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im für das entsprechende Enzym bzw. Protein nicht rekombinanten Mikroorganismnus oder Elternstamm öder ein Gen bzw. Allel verwendet, das für ein entsprechendes Enzym bzw. Protein mit einer niedrigen Aktivität kodiert bzw. das entsprechende Enzym bzw. Protein oder den offenen Leserahmen oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfinderischen Maßnahmen durchgeführt werden.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosaureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182: 842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Weiterhin kann es für die Produktion von L-Aminosäuren, insbesondere L-Threonin vorteilhaft sein, zusätzlich zur Verstärkung des offenen Leserahmens ytfQ, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben verbessert.

Die erfindungsgemäß hergestellten Mikroorganismen können im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert werden. Eine Zusammenfassung über bekannte Kultivierungsmethoden sind im Lehrbuch von Chmiel (Bioprozesstechnik 1. Einführung in die Biover-fahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden. Beispielsweise können Mischungen von Glucose und Fruktose im Verhältnis von ca. 1:1, wie sie in der EP 1 225 230 beschrieben sind, eingesetzt werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung der L-Aminosäure in der Kulturbrühe. Die Kultur wird solange fortgesetzt, bis sich ein Maximum an L-Aminosäuren bzw. L-Threonin gebildet hat. Dieses Ziel wird normalerweise innerhalb von 10 Stunden bis 160 Stunden erreicht.

Aus der entnommenen Kulturbrühe können die L-Aminosäuren gewonnen, gesammelt oder konzentriert und gegebenenfalls gereinigt werden. Typische Methoden zur Reinigung der L-Aminosäuren sind die Ionenaustauschchromatographie und die Kristallisation. Hierdurch erhält man weitgehend reine L-Aminosäuren.

Es ist ebenfalls möglich aus der entnommenen Kulturbrühe (-Fermentationsbrühe) ein Produkt herzustellen, indem man die in der Kulturbrühe enthaltene Biomasse des Bakteriums vollständig (100%) oder nahezu vollständig d.h. mehr als oder größer als (>) 90% entfernt und die übrigen Bestandteile der Fermentationsbrühe weitgehend d.h. zu 30% - 100%, bevorzugt größer gleich (≥) 50%, ≥70% oder ≥90% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10%, kleiner als 5% oder kleiner als 3% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Threonin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pMW218ytfQ

Das ytfQ-Gen aus E. coli K12 wird unter Anwendung der Polymerase-Kettenreaktion (PCR) sowie synthetischen Oligonukleotiden amplifiziert. Ausgehend von der Nukleotidsequenz des ytfQ-Gens in E. coli K12 MG1655 (Accession Number U00096 (Region: 4447985-4448941), Blattner et al. (Science 277: 1453-1474 (1997)) werden PCR-Primer synthetisiert (MWG Biotech, Ebersberg, Deutschland):
ytfQ-1:
   5' - ACCGTAGCCGCATTTTTC - 3' (SEQ ID No. 5)
ytfQ-2:
   5` - AATCGGCATCAGGCATAG - 3' (SEQ ID No. 6)

Die für die PCR eingesetzte chromosomale E. coli K12 MG1655 DNA wird nach Herstellerangaben mit "Qiagen Genomic-tips 100/G" (QIAGEN, Hilden, Deutschland) isoliert. Ein ca. 1152 bp großes DNA-Fragment kann mit den spezifischen Primern unter Standard-PCR-Bedingungen (Innis et al. (1990) PCR Protocols. A Guide to Methods and Applications, Academic Press) mit der Vent-DNA-Polymerase (New England Biolaps GmbH, Frankfurt, Deutschland) amplifiziert werden (SEQ ID No. 7).

Das amplifizierte ytfQ-Fragment wird mit dem Vektor pCR-Blunt II-TOPO (Zero TOPO TA Cloning Kit, Invitrogen, Groningen, Niederlande) den Herstellerangaben entsprechend ligiert und in den E. coli Stamm TOP10 transformiert. Die Selektion Plasmid tragender Zellen erfolgt auf LB Agar, der mit 50 pg/ml Kanamycin versetzt ist. Nach der Plasmid DNA Isolierung wird der Vektor mit den Enzymen PvuI und EcoRI gespalten und nach Überprüfung der Spaltung im 0,8%tigen Agarosegel mit pCRBluntytfQ bezeichnet.

Anschließend wird der Vektor pCRBluntytfQ mit dem Enzym EcoRI gespalten und das ytfQ-Fragment nach Auftrennung im 0,8%tigem Agarosegel aus dem Gel isoliert (QIAquick Gel Extraction Kit, QIAGEN, Hilden, Deutschland) und mit dem low-copy Vektor pMW218 (Nippon Gene, Toyama, Japan), der mit dem Enzym EcoRI verdaut worden ist, ligiert. Der E. coli Stamm DH5α (Grant et al.; Proceedings of the National Academy of Sciences USA, 87 (1990) 4645-4649) wird mit dem Ligationsansatz transformiert und Plasmid tragende Zellen auf LB Agar, der mit 50 pg/ml Kanamycin versetzt ist, selektioniert.

Die erfolgreiche Klonierung kann nach der Plasmid DNA Isolierung durch die Kontrollspaltung mit dem Enzym PvuI nachgewiesen werden.

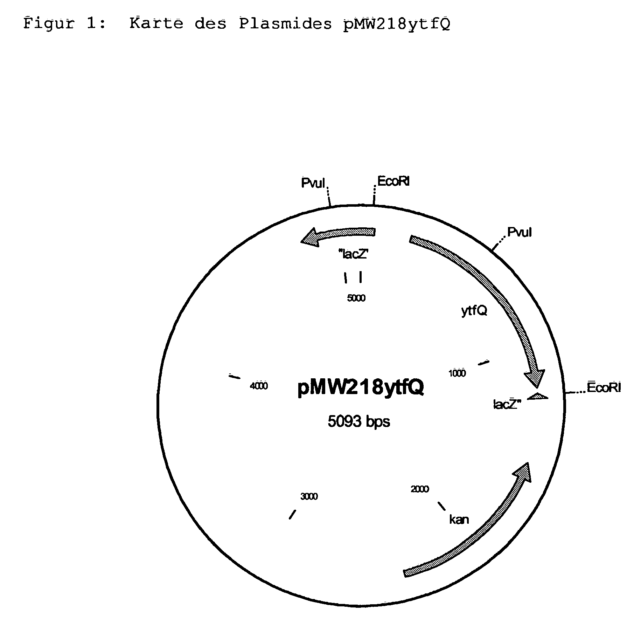

Das Plasmid wird als pMW218ytfQ (Figur 1) bezeichnet.

### Beispiel 2

### Herstellung von L-Threonin mit dem Stamm MG442/pMW218ytfQ

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wird mit dem in Beispiel 1 beschriebenen Expressionsplasmid pMW218ytfQ und mit dem Vektor pMW218 transformiert und auf LB Agar mit 50 µg/ml Kanamycin Plasmid tragende Zellen selektioniert. Auf diese Weise entstehen die Stämme MG442/pMW218ytfQ und MG442/pMW218. Ausgewählte Einzelkolonien werden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Kanamycin, weiter vermehrt. Die Bildung von L-Threonin wird in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Kanamycin, beimpft und für 16 Stunden bei 37°C und 180 rpm auf einem ESR Inkubator der Firma Kühner AG (Birsfelden, Schweiz) inkubiert. Je 250 µl dieser Vorkultur werden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Kanamycin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wird die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442/pMW218 | 6,4 | 2,15 |
| MG442/pMW218ytfQ | 5,5 | 2,6 |

Kurze Beschreibung der Figur:
- Figur 1:: Karte des das ytfQ-Gen enthaltenden Plasmides pMW218ytfQ

Längenangaben sind als ca.-Angaben aufzufassen. Die verwendeten Abkürzungen und Bezeichnungen haben folgende Bedeutung:
- kan: Gen, welches für die Kanamycinresistenz kodiert
- ytfQ: Kodierregion des ytfQ-Gens
- lacZ': Genfragment, welches für das α-Peptid der β-Galaktosidase kodiert

Die Abkürzungen für die Restriktionsenzyme haben folgende Bedeutung:
- EcoRI: Restriktionsendonuklease aus Escherichia coli
- PvuI: Restriktionsendonoklease aus Proteus vulgaris

## Patentansprüche

1. Rekombinante Mikroorganismen, die einen verstärkten oder überexprimierten ytfQ-ORF enthalten, dessen Genprodukt als putatives Bindeprotein eines ATP-abhängigen Zuckertransporters annotiert ist, dessen Aminosäuresequenz zu mindestens 80% identisch ist mit einer Aminosäuresequenz, ausgewählt aus der Gruppe SEQ ID No. 2 und SEQ ID No. 4.

2. Mikroorganismen gemäss Anspruch 1, **dadurch gekennzeichnet, dass** sie ein überexprimiertes oder verstärktes Polynukleotid enthalten ausgewählt aus der Gruppe:
a) Polynukleotid mit einer Nukleotidsequenz, ausgewählt aus SEQ ID No. 1 oder SEQ ID No. 3 und den dazu komplementären Nukleotidsequenzen;
b) Polynukleotid mit einer Nukleotidsequenz, die der SEQ ID No. 1 oder SEQ ID No. 3 im Rahmen der Degeneration des genetischen Codes entspricht;
c) Polynukleotidsequenz mit einer Sequenz, die mit der zur Sequenz SEQ ID No. 1 oder SEQ ID No. 3 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;

3. Mikroorganismen gemäss Anspruch 1, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2 und SEQ ID No. 4.

4. Mikroorganismen gemäss Anspruch 1, **dadurch gekennzeichnet, dass** das Polypeptid die Aminosäuresequenz aufweist, die 100% identisch ist mit einer der Sequenzen, ausgewählt aus der Gruppe SEQ ID No. 2 oder SEQ ID No. 4.

5. Mikroorganismus gemäss den Ansprüchen 1 bis 4, **dadurch gekennzeichnet, dass** sie durch Transformation, Transduktion, Konjugation oder eine Kombination dieser Methoden hergestellt werden, mit einem Vektor, der den ytfQ-ORF, ein Allel dieses ORFs oder Teile davon und/oder einen Promotor enthält.

6. Mikroorganismen gemäss den Ansprüchen 1 bis 5, in denen die Kopienzahl des ytfQ-ORFs oder der Allele um mindestens 1 erhöht vorliegt.

7. Mikroorganismen gemäss Anspruch 6, **dadurch gekennzeichnet, dass** die Erhöhung der Kopienzahl des ytfQ-ORFs um mindestens 1 durch Integration des ORFs oder der Allele in das Chromosom der Mikroorganismen bewirkt wird.

8. Mikroorganismen gemäss Anspruch 6, **dadurch gekennzeichnet, dass** die Erhöhung der Kopienzahl des ytfQ-ORFs um mindestens 1 durch einen extrachromosomal replizierenden Vektor erzielt wird.

9. Mikroorganismen gemäss den Ansprüchen 1 bis 8, **dadurch gekennzeichnet, dass** man zur Erzielung der Verstärkung
a) die Promotor- und Regulationsregion oder die Ribosomenbindungsstelle stromaufwärts des ytfQ-ORFs mutiert, oder
b) Expresssionskassetten oder Promotoren stromaufwärts des ytfQ-ORFs einbaut.

10. Mikroorganismen gemäss den Ansprüchen 1 bis 9, **dadurch gekennzeichnet, dass** die Expression des ytfQ-ORFs unter der Kontrolle eines die Expression des ORFs verstärkenden Promotors steht.

11. Mikroorganismen gemäss den Ansprüchen 1 bis 10, **dadurch gekennzeichnet, dass** durch die Verstärkung des ytfQ-ORFs die Konzentration oder Aktivität des ytfQ-Genproduktes (Proteins) um mindestens 10% erhöht sind, bezogen auf die Aktivität oder Konzentration des Genproduktes im für den ytfQ-ORF nicht rekombinanten Mikroorganismus oder Elternstamm.

12. Mikroorganismen gemäss den Ansprüchen 1 bis 11, **dadurch gekennzeichnet, dass** die Mikroorganismen aus den Gattungen Escherichia, Erwinia, Providencia und Serratia ausgewählt sind.

13. Mikroorganismen gemäss den Ansprüchen 1 bis 12, **dadurch gekennzeichnet, dass** sie L-Threonin produzieren.

14. Verfahren zur Herstellung von L-Threonin durch Fermentation von rekombinanten Mikroorganismen der Familie Enterobacteriaceae, **dadurch gekennzeichnet, dass** man
a) die L-Threonin produzierenden Mikroorganismen gemäß den Ansprüchen 1 bis 13 in einem Medium kultiviert, die gewünschte L-Aminosäure im Medium oder in den Zellen anreichert und
b) L-Threonin isoliert, wobei die Biomasse in ihrer Gesamtheit oder Anteilen (≥ 0 bis 100%) im isolierten Produkt verbleibt oder vollständig entfernt wird.

15. Verfahren gemäss Anspruch 14, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yibD von Escherichia coli,
w) das Genprodukt des offenen Leserahmens (ORF) yjcG von Escherichia coli,
x) das Genprodukt des offenen Leserahmens (ORF) ytfR von Escherichia coli,
y) das Genprodukt des offenen Leserahmens (ORF) ytfT von Escherichia coli, und
z) das Genprodukt des offenen Leserahmens (ORF) yjfF von Escherichia coli
verstärkt, insbesondere überexprimiert.

16. Verfahren gemäss den Ansprüchen 14 und 15, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das Genprodukt des offenen Leserahmens (ORF) yjfA von Escherichia coli,
d) das Genprodukt des offenen Leserahmens (ORF) ytfP von Escherichia coli,
e) das für die Phosphoenolpyruvat-Carboxykinase kodierende pckA-Gen,
f) das für die Pyruvat-Oxidase kodierende poxB-Gen,
g) das für den DgsA-Regulator des Phosphotransferase-Systems kodierende dgsA-Gen,
h) das für den Fructose-Repressor kodierende fruR-Gen,
i) das für den Sigma³⁸-Faktor kodierende rpoS-Gen, und
j) das für die Aspartat Ammonium-Lyase kodierende aspA-Geh
abschwächt, insbesondere ausschaltet oder die Expression verringert.

## Claims

1. Recombinant microorganisms which contain an enhanced or overexpressed ytfQ-ORF, the gene product of which is annotated as being a putative binding protein of an ATP-dependent sugar transporter whose amino acid sequence is at least 80% identical to an amino acid sequence selected from the group SEQ ID No. 2 and SEQ ID No. 4.

2. Microorganisms according to Claim 1, **characterized in that** they contain an overexpressed or enhanced polynucleotide selected from the group:
a) polynucleotide having a nucleotide sequence, selected from SEQ ID No. 1 and SEQ ID No. 3 and the nucleotide sequences complementary thereto;
b) polynucleotide having a nucleotide sequence which corresponds to SEQ ID No. 1 or SEQ ID No. 3 within the limits of the degeneracy of the genetic code;
c) polynucleotide sequence having a sequence which hybridizes, under stringent conditions, with the sequence which is complementary to the sequence SEQ ID No. 1 or SEQ ID No. 3, with the stringent conditions being achieved by means of a washing step in which the temperature extends over a range of from 64°C to 68°C and the salt concentration of the buffer extends over a range of from 2×SSC to 0.1×SSC.

3. Microorganisms according to Claim 1, **characterized in that** the polypeptide possesses an amino acid sequence which is at least 95% identical to one of the sequences selected from the group SEQ ID No. 2 and SEQ ID No. 4.

4. Microorganisms according to Claim 1, **characterized in that** the polypeptide possesses the amino acid sequence which is 100% identical to that of one of the sequences selected from the group consisting of SEQ ID No. 2 and SEQ ID No. 4.

5. Microorganisms according to Claims 1 to 4, **characterized in that** they are produced by transformation, transduction or conjugation, or a combination of these methods, with a vector which contains the ytfQ-ORF, an allele of this ORF, or parts thereof, and/or a promotor.

6. Microorganisms according to Claims 1 to 5, in which the copy number of the ytfQ-ORF or the alleles has been increased by at least 1.

7. Microorganisms according to Claim 6, **characterized in that** the increase in the copy number of the ytfQ-ORF by at least 1 is achieved by integrating the ORF or the alleles into the chromosome of the microorganism.

8. Microorganisms according to Claim 6, **characterized in that** the increase in the copy number of the ytfQ-ORF by at least 1 is achieved by means of a vector which replicates extrachromosomally.

9. Microorganisms according to Claims 1 to 8, **characterized in that**, in order to achieve the potentiation,
a) the promoter and regulatory region or the ribosomal binding site upstream of the ytfQ-ORF is mutated, or
b) expression cassettes or promoters are incorporated upstream of the ytfQ-ORF.

10. Microorganisms according to Claims 1 to 9, **characterized in that** the expression of the ytfQ-ORF is under the control of a promoter enhancing the expression of the ORF.

11. Microorganisms according to Claims 1 to 10, **characterized in that** enhancing the ytfQ-ORF increases the concentration or activity of the ytfQ gene product (protein) by at least 10%, based on the activity or concentration of the gene product in the parent strain or microorganism not recombinant for the ytfQ-ORF.

12. Microorganisms according to Claims 1 to 11, **characterized in that** the microorganisms are selected from the genera Escherichia, Erwinia, Providencia and Serratia.

13. Microorganisms according to Claims 1 to 12, **characterized in that** they produce L-threonine.

14. Process for preparing L-threonine by fermenting recombinant microorganisms of the Enterobacteriaceae family, **characterized in that**
a) the L-threonine-producing microorganisms according to Claims 1 to 13 are cultured in a medium, the desired L-amino acid is enriched in the medium or in the cells, and
b) L-threonine is isolated, with the biomass remaining in its entirety or in portions (from ≥ 0 to 100%) in the isolated product or being removed completely.

15. Process according to Claim 14, **characterized in that**, for the purpose of preparing L-threonine, microorganisms are fermented in which one or more of the genes selected from the group:
a) at least one gene of the thrABC operon encoding aspartate kinase, homoserine dehydrogenase, homoserine kinase and threonine synthase,
b) the pyruvate carboxylase-encoding Corynebacterium glutamicum pyc gene,
c) the phosphoenolpyruvate synthase-encoding pps gene,
d) the phosphoenolpyruvate carboxylase-encoding ppc gene,
e) the pntA and pntB genes encoding the subunits of pyridine transhydrogenase,
f) the rhtC gene encoding the threonine resistance-mediating protein,
g) the threonine export carrier protein-encoding Corynebacterium glutamicum thrE gene,
h) the glutamate dehydrogenase-encoding gdhA gene,
i) the ptsH gene encoding the phosphohistidine protein hexose phosphotransferase,
j) the ptsI gene encoding enzyme I of the phosphotransferase system,
k) the crr gene encoding the glucose-specific IIA component,
l) the ptsG gene encoding the glucose-specific IIBC component,
m) the cysteine synthase A-encoding cysK gene,
n) the cysB gene encoding the regulator of the cys regulon,
o) the cysJ gene encoding the NADPH sulfite reductase flavoprotein,
p) the cysI gene encoding the NADPH sulfite reductase hemoprotein,
q) the adenylyl sulfate reductase-encoding cysH gene,
r) the sucA gene encoding the decarboxylase subunit of 2-ketoglutarate dehydrogenase,
s) the sucB gene encoding the dihydrolipoyltranssuccinase E2 subunit of 2-ketoglutarate dehydrogenase,
t) the sucC gene encoding the β-subunit of succinyl-CoA synthetase,
u) the sucD gene encoding the α-subunit of succinyl-CoA synthetase,
v) the gene product of the Escherichia coli yibD open reading frame (ORF),
w) the gene product of the Escherichia coli yjcG open reading frame (ORF),
x) the gene product of the Escherichia coli ytfR open reading frame (ORF),
y) the gene product of the Escherichia coli ytfT open reading frame (ORF),
z) the gene product of the Escherichia coli yjfF open reading frame (ORF)
is/are additionally, at the same time, enhanced, in particular overexpressed.

16. Process according to Claims 14 and 15, **characterized in that**, for the purpose of preparing L-threonine, microorganisms are fermented in which one or more of the genes selected from the group:
a) the threonine dehydrogenase-encoding tdh gene,
b) the malate dehydrogenase-encoding mdh gene,
c) the gene product of the Escherichia coli yjfA open reading frame (ORF),
d) the gene product of the Escherichia coli ytfP open reading frame (ORF),
e) the pckA gene encoding the phosphoenolpyruvate carboxykinase,
f) the pyruvate oxidase-encoding poxB gene,
g) the dgsA gene encoding the DgsA regulator of the phosphotransferase system,
h) the fruR gene encoding the fructose repressor,
i) the rpoS gene encoding the sigma³⁸ factor, and,
j) the aspartate ammonium lyase-encoding aspA gene,
is/are additionally, at the same time, attenuated, in particular eliminated, or their expression is reduced.

## Revendications

1. Microorganismes recombinants qui contiennent un cadre de lecture ouvert ytfQ renforcé ou surexprimé, dont le produit génique est annoté comme une protéine de liaison putative d'un transporteur de sucre dépendant de l'ATP, dont la séquence d'aminoacides est identique à raison d'au moins 80% à une séquence d'aminoacides choisie dans le groupe de séquences SEQ ID No. 2 et SEQ ID No. 4.

2. Microorganismes selon la revendication 1, **caractérisés en ce qu'**ils contiennent un polynucléotide surexprimé ou renforcé choisi dans le groupe :
a) polynucléotide présentant une séquence nucléotidique choisie parmi les séquences SEQ ID No 1 ou SEQ ID No 3 et les séquences nucléotidiques complémentaires à celles-ci ;
b) polynucléotide présentant une séquence nucléotidique qui correspond aux séquences SEQ ID No 1 ou SEQ ID No 3 dans le cadre de la dégénérescence du code génétique ;
c) séquence polynucléotidique avec une séquence qui s'hybride dans des conditions stringentes avec la séquence complémentaire à la séquence SEQ ID No 1 ou SEQ ID No 3, où les conditions stringentes sont obtenues par une étape de lavage dans laquelle la température s'étend sur une plage de 64°C à 68°C et la concentration en sel du tampon s'étend sur une plage de 2xSSC à 0,1xSSC.

3. Microorganismes selon la revendication 1, **caractérisés en ce que** le polypeptide présente une séquence d'aminoacides qui est identique à raison d'au moins 95% à une des séquences choisies dans le groupe des séquences SEQ ID No 2 et SEQ ID No 4.

4. Microorganismes selon la revendication 1, **caractérisés en ce que** le polypeptide présente la séquence d'aminoacides qui est identique à raison de 100% à une des séquences choisies dans le groupe des séquences SEQ ID No 2 ou SEQ ID No 4.

5. Microorganismes selon les revendications 1 à 4, **caractérisés en ce qu'**ils sont obtenus par transformation, transduction, conjugaison ou une combinaison de ces procédés avec un vecteur qui contient le cadre de lecture ouvert ytfQ, un allèle de ce cadre de lecture ouvert ou des parties de celui-ci et/ou un promoteur.

6. Microorganismes selon les revendications 1 à 5, dans lesquels le nombre de copies du cadre de lecture ouvert ytfQ ou des allèles se trouve en un nombre augmenté d'au moins une unité.

7. Microorganismes selon la revendication 6, **caractérisés en ce que** l'augmentation du nombre de copies du cadre de lecture ouvert ytfQ d'au moins une unité est obtenue par l'intégration du cadre de lecture ouvert ou des allèles dans le chromosome des microorganismes.

8. Microorganismes selon la revendication 6, **caractérisés en ce que** l'augmentation du nombre de copies du cadre de lecture ouvert ytfQ d'au moins une unité est obtenue par un vecteur à réplication extrachromosomique.

9. Microorganismes selon les revendications 1 à 8, **caractérisés en ce que** pour obtenir le renforcement
a) on mute la région du promoteur et de régulation ou le site de liaison aux ribosomes en amont du cadre de lecture ouvert ytfQ, ou
b) on incorpore des cassettes d'expression ou des promoteurs en amont du cadre de lecture ouvert ytfQ.

10. Microorganismes selon les revendications 1 à 9, **caractérisés en ce que** l'expression du cadre de lecture ouvert ytfQ est sous le contrôle d'un promoteur renforçant l'expression du cadre de lecture ouvert.

11. Microorganismes selon les revendications 1 à 10, **caractérisés en ce que** par le renforcement du cadre de lecture ouvert ytfQ, la concentration ou l'activité du produit génique d'ytfQ (protéine) est augmentée d'au moins 10% par rapport à l'activité ou à la concentration du produit génique dans le microorganisme non recombinant pour le cadre de lecture ouvert ytfQ ou la souche parente.

12. Microorganismes selon les revendications 1 à 11, **caractérisés en ce que** les microorganismes sont choisis parmi les genres Escherichia, Erwinia, Providencia et Sefratia.

13. Microorganismes selon les revendications 1 à 12, **caractérisés en ce qu'**ils produisent de la L-thréonine.

14. Procédé pour la production de L-thréonine par fermentation de microorganismes recombinants de la famille des Enterobacteriaceae, **caractérisé en ce que**
a) on cultive les microorganismes produisant la L-thréonine selon les revendications 1 à 13 dans un milieu, on enrichit le L-aminoacide souhaité dans le milieu ou dans les cellules et
b) on isole la L-thréonine, la biomasse restant dans sa totalité ou en partie (≥ 0 à 100%) dans le produit isolé ou étant complètement éliminée.

15. Procédé selon la revendication 14, **caractérisé en ce que** pour la production de L-thréonine, on fermente des microorganismes dans lesquels en outre on renforce, en particulier surexprime, simultanément un ou plusieurs des gènes choisis dans le groupe :
a) au moins un gène de l'opéron thrABC codant pour l'aspartate-kinase, l'homosérine-déshydrogénase, l'homosérine-kinase et la thréonine-synthase,
b) le gène pyc codant pour la pyruvate-carboxylase de Corynebacterium glutamicum,
c) le gène pps codant pour la phosphoénol-pyruvate-synthase,
d) le gène ppc codant pour la phosphoénol-pyruvate-carboxylase,
e) les gènes pntA et pntB codant pour les sous-unités de la pyridine-transhydrogénase,
f) le gène rhtC codant pour la protéine conférant une résistance à la thréonine,
g) le gène thrE codant pour la protéine thréonine-export-carrier de Corynebacterium glutamicum,
h) le gène gdhA codant pour la glutamate-déshydrogénase,
i) le gène ptsH codant pour la phosphohistidine-protéine-hexose-phosphotransférase,
j) le gène ptsI codant pour l'enzyme I du système de phosphotransférase,
k) le gène crr codant pour le composant IIA spécifique du glucose,
l) le gène ptsG codant pour le composant IIBC spécifique du glucose,
m) le gène cysK codant pour la cystéine-synthase A,
n) le gène cysB codant pour le régulateur du cys-régulon,
o) le gène cysJ codant pour la flavoprotéine de la NADPH-sulfite-réductase,
p) le gène cysI codant pour l'hémoprotéine de la NADPH-sulfite-réductase,
q) le gène cysH codant pour l'adénylylsulfate-réductase,
r) le gène sucA codant pour la sous-unité décarboxylase de la 2-cétoglutarate-déshydrogénase,
s) le gène sucB codant pour la sous-unité dihydrolipoyltranssuccinase E2 de la 2-cétoglutarate déshydrogénase,
t) le gène sucC codant pour la sous-unité β de la succinyl-CoA synthétase,
u) le gène sucD codant pour la sous-unité α de la succinyl-CoA synthétase,
v) le produit génique du cadre de lecture ouvert (ORF) yibD d'Escherichia coli,
w) le produit génique du cadre de lecture ouvert (ORF) yjcG d'Escherichia coli,
x) le produit génique du cadre de lecture ouvert (ORF) ytfR d'Escherichia coli,
y) le produit génique du cadre de lecture ouvert (ORF) ytfT d'Escherichia coli, et
z) le produit génique du cadre de lecture ouvert (ORF) yjfF d' Escherichia coli.

16. Procédé selon les revendications 14 et 15, **caractérisé en ce que** pour la production de L-thréonine, on fermente des microorganismes dans lesquels, en outre, on affaiblit, en particulier supprime ou diminue simultanément l'expression d'un ou de plusieurs des gènes choisis dans le groupe :
a) le gène tdh codant pour la thréonine-déshydrogénase,
b) le gène mdh codant pour la malate-déshydrogénase,
c) le produit génique du cadre de lecture ouvert (ORF) yjfA d'Escherichia coli.
d) le produit génique du cadre de lecture ouvert (ORF) ytfP d'Escherichia coli.
e) le gène pckA codant pour la phosphoénolpyruvate-carboxykinase,
f) le gène poxB codant pour la pyruvate-oxydase,
g) le gène dgsA codant pour le régulateur DgsA du système de phosphotransférase,
h) le gène fruR codant pour le répresseur du fructose,
i) le gène rpoS codant pour le facteur Sigma³⁸, et
j) le gène aspA codant pour l'aspartate ammonium-lyase.
